# EUROPEAN PATENT SPECIFICATION

(11) **EP 4 340 634 B1**
(45) Date of publication and mention of the grant of the patent: **19.08.2026**
(21) Application number: 22729236.4
(22) Date of filing: 17.05.2022
(51) Int. Cl.: A23K 20/28, A23K 50/80

(54) **SILICIC ACID IN AQUACULTURE**
KIESELSÄURE IN AQUAKULTUR
ACIDE SILICIQUE EN AQUACULTURE

(30) Priority: 19.05.2021 EP 21174738
(43) Date of publication of application: 27.03.2024
(73) Proprietor: Barlaa B.V., 1398 AB Muiden (NL)
(72) Inventor: LAANE, Henk Maarten, 1398 AB Muiden (NL); VAN STEE, Cornelis Hendrik Geuvel, 3344 BP Hendrik Ido Ambacht (NL)
(74) Representative: Nederlandsch Octrooibureau
(86) International application number: PCT/EP2022/063347
(87) International publication number: WO 2022/243331

(56) References cited:
- WO-A1-03/101915
- WO-A1-2011/071379
- WO-A1-2015/041531
- WO-A1-2021/005860
- VAN DYCK K ET AL: "Bioavailability of silicon from food and food supplements", FRESENIUS' JOURNAL OF ANALYTICAL CHEMISTRY, SPRINGER, DE, vol. 363, no. 5-6, 1 March 1999 (1999-03-01), pages 541 - 544, XP002633342, ISSN: 0937-0633, DOI: 10.1007/S002160051243

## Description

### Field of the Invention

The present invention concerns the field of aquaculture or aquafarming, such as farming of fish, crustaceans and mollusks. More in particular, the present invention relates to the use of bioavailable silicic acid compounds in aquaculture, wherein the use has the objective of enhancing growth of the aquatic animal; accelerating growth of the aquatic animal; increasing the weight of the aquatic animal; reducing the time to harvest; improving feed utilization; and/or increasing the feed conversion rate. The present disclosure also relates to aquafarming methods wherein said bioavailable silicic acid compound is used, as well as to specific compositions comprising bioavailable silicic acid compounds, which are specifically adapted for use in aquaculture.

### Background of the Invention

Ending malnutrition and achieving food security are global priorities. Within this context, the importance of fisheries in local and global food systems and its contribution to nutrition and health, particularly for the poor, can hardly be overstated. Capture fisheries and aquaculture play complementary roles in ascertaining fish availability and access.

In 2018, FAO estimated that global fish production reached about 171 million tonnes in 2016, with aquaculture representing 47 percent of this total (and 53 percent if non-food uses, including reduction to fishmeal and fish oil, are excluded). Hence, with capture fishery production relatively static since the late 1980s, aquaculture is responsible for most of the growth in the supply of fish for human consumption. With worldwide nutrition and health needs on the rise and with its growing role in meeting these needs, aquaculture has the potential to become much more harmful to the environment than capture fishery. Over the past years it has become increasingly apparent that the success and continued expansion of the fish farming sector is highly dependent on technological development. Technological developments relating to pond preparation, species selection, stocking density, water exchange and the application of feed, fertilizers and biostimulants have attracted particular attention. One particularly important aspect is the development of high-quality feed, fertilizer and/or biostimulant products that enable significant (further) increases in aquaculture productivity and do so without negative impact on the environment. More importantly, perhaps, the production and/or use of such products should not compete for systemic resources with food availability.

As in terrestrial animals, protein plays a vital role in fish and other aquatic species. It constitutes about 65 - 75% of fish body weight (on dry matter basis). Fish require protein for growth, development and reproduction. Protein deficient feeds can negatively affect growth or lead to interruption of growth and loss of weight. Feed cost constitutes the major portion of the variable cost in fish farming and protein is the most expensive feed ingredient. Therefore cost effective feed composition that can satisfy nutritional requirements and feed management that can optimize yield is crucial. To support fish production, compound aqua feeds are formulated to meet the known nutrients requirements for protein, lipids, carbohydrates, vitamins and minerals and to contain functional materials other than nutrients such as attractants, anti-oxidants, immunostimulants, enzymes, pigments, organic acids, prebiotics, probiotics, feeding stimulants, biostimulants antibiotics, and hormones. Attempts have been made to develop fish diets with reduced protein levels without compromising growth performance. In US 2021/0068426, for example, a fish diet is disclosed providing a sub-optimal dose of protein in combination with butyric acid. According to US 2021/0068426, experimental results obtained with the diet support a role for butyrate in increasing protein absorption by affecting intestinal morphology and/or in increasing taurine absorption into the tissues, probably by increasing transcription of the taurine transporter.

Another common approach to try to optimize yields in aquaculture is pond fertilization to boost natural feed. Algae constitute a natural feed source for many types of fish (and other aquatic species) and the contribution of algae to growth of fish is substantial. The two most common (inorganic) fertilizers used worldwide in aquaculture are triple superphosphate (TSP) and urea. With fertilization, it is important to control the algae load in the water of ponds to stay within optimal levels with a view to dissolved oxygen content (DO). In flowing rivers DO is usually not an issue, due to ample water movement. In highly eutrophic fish ponds (or lakes) algae bloom and hypoxia can readily become a problem.

DO is one of the main limiting environmental variables that affect fish performance. Low DO affects feed intake negatively and reduces digestibility. At high DO, feed assimilation is improved, which may be due to improved blood flow to the gastrointestinal tract and lower energy cost of feed digestion and absorption of nutrients. Therefore, more energy is available for growth. Nile tilapia have been shown to perform significantly less in terms of final body weight, specific growth rate and feed conversion rate (FCR) under hypoxia compared to normoxia (5 mg/L which is 50% of saturation). Hypoxia negatively affected intestinal morphology. In non-aerated ponds, DO levels fluctuate during the day and will be somewhere 0 - 15 mg/L with the highest values in the afternoon and the lowest values just before sunrise. Pond aeration keeps DO at an acceptable level with minimal fluctuations. In practice, however, DO is often beyond control in many small-scale farms where aeration for fishponds is not available or too expensive.

Another environmental variable that affect fish performance is water pH. Previous research has suggested that increasing pH by one unit from approximately 6.5 to 7.5 can improve FCR (feed conversion rate) by about 0.5 unit and TGC (thermal growth coefficient) by 0.2 unit, respectively. Un-ionized ammonia, which is toxic to fish, increases with increasing pH and water temperature. Therefore, in order to achieve best results pH should be maintained between 7 and 8. Among the environmental factors pH is relatively easy to manage (with a view to growth optimization). Small-scale farmers typically manage water pH using lime. Nitrogen fertilizers are a source of acidity in ponds.

As will be understood there is still an unmet need for providing new modalities in aquaculture that can, in particular, improve productivity. It is the object of the present invention to satisfy this need.

### Summary of the Invention

Generally stated, the present invention resides in the finding that the aforesaid objective can be realized by using bioavailable forms of silicic acid. Numerous experiments, some of which are described in the experimental section here below, have shown that the use of certain bioavailable silicic acid compounds in aquaculture has a remarkable, beneficial impact on productivity, water quality and/or environmental impact, such as increased growth of the aquatic species, increased feed conversion ratio, increased zooplankton and phytoplankton levels, improved dissolved oxygen content, favorable pH values, lower N (ammonia) levels, etc. These remarkable beneficial effects observed are probably intertwined and interdependent and the various mechanisms potentially involved have not yet been elucidated in full.

WO 03/101915 describes the treatment of rainbow trout with non-colloidal silicic acid and boric acid in order to protect the fish against pathology associated with Saprolegnia infection. The treatment was started as soon as the first symptoms of Saprolegnia infection appeared. According to WO 03/101915 the treatment restored the immunological status of the fish and protected the fish from dying. Based on these results, WO 03/101915 teaches to employ the non-colloidal silicic acid and boron containing solutions for strengthening fish and increasing their resistance against microbial infection. WO 03/101915 does not teach any other effects of the treatment on productivity, water or pond quality and/or environmental impact in aquaculture.

WO 2015/041531 concerns processes for producing diatom cultures with improved storage compound production capability, by subjecting a starter culture to selective pressure, thus giving a competitive advantage to storage compound producing species of diatoms, by subjecting said starting culture, under conditions favorable for diatom dominance, to a cycle of alternating dark phases and light phases and providing limitation of availability of at least one essential growth nutrient, typically nitrogen, in one or more of said light phases, whereby non-limiting bioavailable silicon concentrations are present at least in the dark phases. WO 2015/041531 teaches that silicon is specifically used to build the diatom cell wall and that in case of depletion cells stop their division process but continue to produce storage compounds in an improved rate (as compared to a situation where other compounds than bioavailable silicon are limited. WO 2015/041531 does not provide any teaching or suggestion to the effect that the mere addition of bioavailable silicon to conventional fish framing ponds, will enhance growth of the fish and it neither teaches or suggests the use of a bioavailable silicic acid compound.

Hence, to the inventors' best knowledge, they have been the first to show that the addition of bioavailable silicic acid compounds favorably affects productivity, water quality and/or environmental impact of aquaculture, in particular that it increases growth of the aquatic species, increases feed conversion ratio, increases zooplankton and phytoplankton levels, improves dissolved oxygen content, favorably affects pH values, and lowers N (ammonia) levels, among other things.

Hence, a first aspect of the invention concerns the use of compositions comprising a bioavailable silicic acid compound in the farming of aquatic animals selected from the group consisting of fish, crustaceans and/or mollusks, wherein the use has the objective of and/or results in one or more of:
- enhancing growth of the aquatic animal;
- accelerating growth of the aquatic animal;
- increasing the weight of the aquatic animal;
- reducing the time to harvest;
- improving feed utilization; and
- increasing the feed conversion rate.

Also disclosed herein is a method of farming aquatic animals selected from fish, crustaceans and/or mollusks, said method comprising the step of treating the aquatic animals with a composition comprising a bioavailable silicic acid compound.

Also disclosed herein is a method of farming aquatic animals selected from fish, crustaceans and/or mollusks, said method comprising the step of treating the water in which the aquatic animals are kept with a composition comprising a bioavailable silicic acid compound.

Also disclosed herein is a non-therapeutic method of farming aquatic animals selected from fish, crustaceans and/or mollusks, said method comprising the step of treating the aquatic animals and/or the water they are kept in with a composition comprising a bioavailable silicic acid compound.

Also disclosed herein is a composition comprising a bioavailable silicic acid compound for use in a method of treating aquatic animals selected from the group consisting of fish, crustaceans and/or mollusks.

Also disclosed herein is a composition comprising a bioavailable silicic acid compound for use in the manufacture of a product for treating aquatic animals selected from the group consisting of fish, crustaceans and/or mollusks.

Also disclosed herein is a method of treating aquatic animals selected from the group consisting of fish, crustaceans and/or mollusks by administering to the aquatic animals a composition comprising a bioavailable silicic acid compounds.

The invention as well as the preferred embodiments thereof will become apparent to those skilled in the art, based on the following detailed description and examples.

### Detailed description of the Invention

As will be apparent to those skilled in the art, based on the present teachings, the compositions used in accordance with the invention comprise a bioavailable silicic acid compound.

In the context of the present invention, the term 'silicic acid' is used to refer to compounds with the basic structure [SiO₂₋ₓ(OH)₂ₓ(H₂O)ₘ]ₙ, wherein x = 0 or 1; m = 0, 1 or 2; and n ≥ 1. Such compounds thus comprise orthosilicic acid (Si(OH)₄) as the fundamental building block. Si(OH)₄ is a relatively unstable and tends to undergo autocondensation into dimers (2Si(OH)₄ ↔ (HO)₃Si-O-Si(OH)₃ + H₂O), trimers ((HO)₃Si-O-Si(OH)₃+ Si(OH)₄ ↔ (HO)₃Si-O-Si(OH)₂-O-Si(OH)₃ + H₂O), etc., to form oligomers and/or polymers. The formation of small-size particles (non-colloids, sub-colloids and micro-colloids, colloids) is a gradual process. This process eventually results in the formation of a soft gel, which is poorly bioavailable. The formation of colloids and gels is pH dependent. The longest gelling time occurs at pH 2. At lower and more alkaline pH, the time for colloid and finally gel formation decreases (Ralph K. Iler. The Chemistry of Silica. Wiley: New York, 1979). The stages from monomer to sol-gel polymerization can be summarized as follows:
1 . monomeric orthosilicic acid in acid medium;
2. polymerization of orthosilicic acid, from monomers into dimers, trimers, tetramers, linear or cyclic oligomers up to structures of more than thousand silicon molecules;
3. further condensation into linear or randomly branched polymers, which typically take the form of small spherical particles, having a particle size of between 1-10 nm, referred to as 'subcolloidal', consisting of several thousands of silicic acid monomers;
4. growth of these particles to a particle size of about 10-100 nm, referred to as colloidal;
5. linking of particles into chains (aggregation);
6. chained into network and extension throughout the liquid (aggregation, pre-gel);
7. thickening into a gel.

The term 'bioavailable' as used in the context of the present invention refers to silicic acid provided in a form that may enter into living organisms. Bioavailable forms of silicic acid include, in particular, monomeric silicic acid (also referred to as orthosilicic acid) as well as dimeric silicic acid, which is believed to exist in equilibrium with monomeric silicic acid in aqueous systems.

The term 'bioavailable silicic acid compound', is used herein to embrace compounds with the basic structure [SiO₂₋ₓ(OH)₂ₓ(H₂O)ₘ]ₙ that are in a form capable of releasing/liberating monomeric silicic acid (i.e. by depolymerization reactions), e.g. when dispersed in water or an aqueous system. Such bioavailable silicic acid compounds include, in particular, the stage 2 and 3 compounds as defined here above, in addition to monomeric silicic acid (also referred to as orthosilicic acid) and dimeric silicic acid. Hence, in preferred embodiments of the invention, the bioavailable silicic acid compound is selected from the group consisting of monomeric silicic acid (also referred to as orthosilicic acid), dimeric silicic acid, oligomeric silicic acid and polymeric silicic acid in subcolloidal form and combinations thereof.

Preferably, in the compositions employed in accordance with the present invention, at least 50 mol.% of the silicon contained in the composition is in the form of a bioavailable silicic acid compound as defined herein, more preferably at least 60 mol.%, still more preferably at least 70 mol.%, still more preferably at least 75 mol.%, still more preferably at least 80 mol.%, still more preferably at least 85 mol.%, still more preferably at least 90 mol.%, still more preferably at least 95 mol.%, still more preferably at least 97.5 mol.%.

The composition employed in accordance with the present invention, preferably comprise subcolloidal silicic acid, i.e. silicic acid that is mainly in stages 2 and 3 as defined here above. Solutions comprising such subcolloidal particles passes through a 0.1 micron filter. Though the monomer might be present (due to the equilibrium), preferably no measurable free orthosilicic acid is present. The invention is not directed to uses of and/or methods employing silicic acid in colloidal form or in sol form. Although minor amounts of these species may be present in the compositions of the invention, the compositions of the invention substantially comprises non-colloidal silicic acid (i.e. compounds with the basic structure [SiO₂₋ₓ(OH)₂ₓ(H₂O)ₘ]ₙ that are mainly in stage 2 and stage 3, as described above).

In particularly preferred embodiments of the invention, the bioavailable silicic acid compound used is subcolloidal silicic acid, more preferably silicic acid in the form of subcolloidal particles having a size within the range of 1-10 nm, more preferably within the range of 1.5-8 nm, still more preferably 2-6 nm, still more preferably 3-5 nm, most preferably 3.5-4 nm. Particle size determinations can be made using ²⁹Si NMR spectroscopy, TEM and/or SEM. In preferred embodiments of the invention, at least 50 % of the silicic acid containing particles in the compositions have a particle diameter within the aforementioned size ranges, more preferably at least 60 %, still more preferably at least 70 %, still more preferably at least 75 %, still more preferably at least 80 %, still more preferably at least 85 %, still more preferably at least 90 %, most preferably at least 95 %.

The composition employed in accordance with the present invention, typically have the form of aqueous dispersions or solutions of the bioavailable silicic acid compound at adequate concentrations that can be added to the water in which the aquatic species is kept in a practical manner. Although the invention is not particularly limited in this regard, preferred embodiments are envisaged wherein the composition as employed comprises bioavailable silicic acid compounds at a level of at least 0.001 ppm, e.g. at least 0.005 ppm, at least 0.01 ppm, at least 0.05 ppm, at least 0.1 ppm, at least 0.5 ppm, at least 1 ppm, at least 5 ppm, or at least 10 ppm. Furthermore, preferred embodiments are envisaged wherein the composition as employed comprises bioavailable silicic compounds at a level below 5000 ppm, e.g. below 1000 ppm, below 500 ppm, below 100 ppm, below 50 ppm or below 10 ppm.

The afore defined aqueous solutions or dispersions may typically be produced from a highly concentrated aqueous product or a product in dry solid form, i.e. by diluting/mixing such a product with an adequate quantity of water or with an adequate quantity of feed just before actual use. It is known that aggregation of subcolloidal silicic acid particles (into the forms of stage 4 or higher) may occur over time, especially in the case of highly concentrated products, resulting in opalescence, turbidity, light reflection, colloid and gel formation and thus loss of bioactivity upon storage. Hence, such products in concentrated or dry solid form may contain additives effective in preventing formation of colloidal or macrocolloidal silicic acid particles. International patent application no. WO 2003/101915 and international patent application no. WO 2011/071379, both incorporated herein by reference, describe various techniques to stabilize concentrated products comprising bioavailable silicic acid compounds. Hence, the compositions employed in accordance with the present invention may contain additives, such as those taught by WO 2003/101915 and WO 2011/071379. As will be understood by those skilled in the art though, based on the present teachings, the presence of such additives in the compositions employed per se is not critical or essential for attaining the beneficial effects in aquaculture (such as increased growth, increased feed conversion ratio, increased zooplankton and phytoplankton levels, improved dissolved oxygen content, favorable pH values, lower N (ammonia) levels, etc.); what counts is that the composition employed contains bioavailable silicic acid compounds, irrespective of how it is made and provided and/or what measures may have been taken to stabilize it during (prolonged) storage. Notwithstanding the former, from a practical standpoint, the compositions taught by WO 2003/101915 and WO 2011/071379 may have advantages for the purposes of the present invention. Hence, in certain preferred embodiments of the invention, the composition comprises an acidified aqueous solution of (1) subcolloidal silicic acid in combination with (2) boric acid and/or (3) a water absorbing additive. In preferred embodiments, said water absorbing additive comprises a humectant selected from the group consisting of a polysorbate, a vegetable gum, a substituted cellulose, a polyglycerol ester of a fatty acid, a polyethylene glycol, a polydextrose, a propylene glycol, a propylene glycol alginate, a polyoxyethylene fatty acid ester, a pectine or amidated pectine, a sucrose ester of a fatty acid, an acetylated or hydroxypropyl starch, a starch phosphate, urea, sorbitol, malitol, (pro-)vitamins, and a mixture of two or more of such humectants. Preferably, the water absorbing additive concentration is at least 10 wt.% of the composition, based on dry solids weight, such as at least 25 wt.%, at least 40 wt.% or at least 50 wt.%. The water absorbing additive concentration is typically less than 75 wt.% of the composition, based on dry solids weight, e.g. less than 70 wt.%, less than 65 wt.% or less than 60 wt.%. In embodiments where the bioavailable silicic acid compound is combined with boric acid, preferably the molar Si/B ratio is in the range of 0.1-1000, more preferably 0.5-500, 1-400 or 1.5-300. In preferred embodiments, the composition is filterable through a 0.1 micron filter. In preferred embodiments, the composition is filterable through a 20,000 Mw (Da) filter.

In preferred embodiments of the invention, the composition used may further comprise one or more additional nutrients selected from the group consisting of zinc, manganese, copper, molybdenum, selenium, a humic acid, a fulvic acid and an amino acid. In further preferred embodiments of the invention, the composition may comprise one or more additional fertilizer compounds conventionally used in aquaculture, such as one or more fertilizer compounds selected from the group consisting of urea, ammonium nitrate, ammonium sulfate, calcium nitrate, sodium nitrate, diammonium phosphate, monoammonium phosphate, superphosphate, triple superphosphate, ammonium polyphosphate, potassium nitrate and potassium chloride.

An aspect of the present disclosure concerns the compositions adapted for the uses and methods of the present invention *per* se, e.g. any of the compositions as defined here above, including the concentrated and dry solid form products that need to be mixed/diluted with water prior to actual use. The product may be provided in the form of a container comprising a composition as defined here above, which may be a concentrated product or dry solid form product that needs to be mixed/diluted with water prior to actual use, wherein said container is provided with instructions printed on the container and/or instructions printed on a label provided with the container, to use the composition for the purposes and/or in the manners as defined herein.

As will be understood by those skilled in the art, based on the present teachings, the uses of the present invention entail the addition of the composition comprising bioavailable silicic acid compounds, preferably a composition as defined herein before, to water in which aquatic animals are kept, so as to attain one or more of the beneficial effects mentioned herein (such as increased growth, increased feed conversion ratio, increased zooplankton and phytoplankton levels, improved dissolved oxygen content, favorable pH values, lower N (ammonia) levels, etc.). In accordance with the invention, the compositions comprising bioavailable silicic acid compounds can be added to the water separately. Embodiments are also envisaged though, wherein the composition is mixed or blended with other products added to the water in typical aquafarming practice, such as other fertilizer products, feed, etc. The invention is not particularly limited in this regard. Nonetheless, in a preferred embodiment of the invention, the composition comprising bioavailable silicic acid compounds is added to and/or blended with a feed composition before the feed composition is added to the water. Without wishing to be bound by any theory, it is hypothesized that addition of the composition comprising bioavailable silicic acid compounds to the feed enhances the uptake of bioavailable silicic acid by the aquatic animals, which may be more important in some embodiments than others.

For optimal results the uses entail the addition of the composition comprising bioavailable silicic acid compounds to the water in quantities resulting in level of at least 0.1 ppm of bioavailable silicic acid compound in the water, preferably at least 0.5 ppm, at least 1 ppm, at least 2.5 ppm, at least 5 ppm or at least 10 ppm, e.g. about 25 ppm of bioavailable silicic acid compound in the water. Furthermore, in preferred embodiments of the invention, the uses entail the addition of the composition to the water in quantities resulting in a level of less than 1000 ppm of bioavailable silicic acid compound in the water, preferably less than 750 ppm, less than 500 ppm , less than 250 ppm, less than 100 ppm or less than 50 ppm. Furthermore, with a view to optimal results, the uses entail the repeated addition of the composition to the water in the quantities recited here above, e.g. once every 10 days, preferably once every 14 days, once every 10 days, once every 7 days, once every 5 days, once every 3 days, once every other day, once every day or twice a day.

In other preferred embodiments of the invention, the uses entail the addition of the composition comprising bioavailable silicic acid compounds to the feed, i.e. the/a standard feed composition conventionally used in the farming of the species in suit, in quantities resulting in level of at least 0.001 ppm of bioavailable silicic acid compound in the feed, preferably at least 0.005 ppm, at least 0.01 ppm, at least 0.025 ppm, at least 0.05 ppm or at least 0.10 ppm, e.g. about 0.25 ppm. Furthermore, in preferred embodiments of the invention, the uses entail the addition of the composition to the feed in quantities resulting in a level of less than 10 ppm of bioavailable silicic acid compound in the feed, preferably less than 7.5 ppm, less than 5 ppm , less than 2.5 ppm, less than 1 ppm or less than 0.5 ppm. With a view to optimal results, it is preferred that feed enriched with the bioavailable silicic acid compounds is given to the aquatic animal repeatedly, e.g. the uses entail the repeated administration of feed addition of the composition to the feed in the quantities recited here above, e.g. once every 10 days, once every 7 days, once every 5 days, once every 3 days, once every other day or once every day, two times a day or three times a day.

In preferred embodiments of the invention addition of the composition comprising bioavailable silicic acid compounds to the water and/or to the feed is according to the regimens defined above, is continued for a period of at least 1 week, at least two weeks, at least three week, at least four weeks, at least one month, at least two months, at least three months or at least four months. In preferred embodiments of the invention, addition of the composition comprising bioavailable silicic acid compounds to the water and/or to the feed is according to the regimens defined above, is carried out substantially or entirely throughout the life span or life cycle of the aquatic animals.

In a preferred embodiment of the invention the uses of the present invention entail the addition of the composition to the water in a regimen that is adequate to keep the level of bioavailable silicic acid compound in the water at a level of at least 0.01 ppm, at least 0.05 ppm, at least 0.1 ppm, at least 0.25 ppm, at least 0.5 ppm, at least 1 ppm, or at least 2.5 ppm, substantially throughout the treatment period or during the entire treatment period.

As explained herein before, and as illustrated in the experimental part, the use of bioavailable silicic acid compounds has been found to lead to beneficial results in many different settings, i.e. in different aquatic species, different geographical areas, different water qualities, etc. Hence, the present invention is not particularly limited in this regard. Nonetheless, in particularly preferred embodiment of the invention the uses entail the addition of the compositions comprising bioavailable silicic acid compounds to tanks, ponds or lakes comprising brackish water, fresh water as well as salt water. Embodiments are also envisaged wherein the uses of the invention are applied in the context of aquaculture in open waters, in which case aquatic animals are typically contained in cages placed in the open water, such as in off-shore fish farming. As will be understood by those skilled in the art, based on the present teachings, in such embodiments, the uses may entail the addition of the compositions comprising bioavailable silicic acid compounds to the cages placed in the open water, preferably via the feed, as explained herein elsewhere, so as to avoid the quick leaching away of the silicic acid.

As mentioned herein before, the aquatic species is selected from fish, crustaceans and mollusks. In preferred embodiments of the invention, the aquatic species is selected from the group consisting of fish, in particular from the group of fish species belonging to one of the following families: Acipenseridae Osteoglossidae, Anguillidae, Chanidae, Cyprinidae, Cobitidae, Catastomidae, Curimatidae, Characidae, Ictaluridae, Bagridae, Siluridae, Pangasiidae, Clariidae, Pimelodidae, Callichthyidae, Esocidae, Plecoglossidae, Salmonidae, Gadidae, Atherinidae, Synbranchidae, Centropomidae, Percichthyidae, Moronidae, Serranidae, Terapontidae, Centrarchidae, Percidae, Pomatomidae, Carangidae, Lutjanidae, Sparidae, Sciaenidae, Cichlidae, Mugilidae, Eleotridae, Siganidae, Scombridae, Anabantidae, Belontiidae, Helostomatidae, Osphronemidae, Channidae, Scophthalmidae, Paralichthyidae and Soleidae, such as from the group of fish species consisting of *Acipenser baeri, Acipenser ruthenus, Acipenser stellatus, Acipenser transmontanus, Huso huso, Arapaima gigas, Heterotis niloticus, Anguilla anguilla, Anguilla japonica, Anguilla rostrata, Chanos chanos, Abramis brama, Aspius aspius, Catla catla, Carassius auratus, Carassius carassius, Cirrhinus molitorella, Cirrhinus mrigala, Ctenopharyngodon idellus, Cyprinus carpio, Hypophthalmichthys molitrix, Hypophthalmichthys nobilis, Labeo calbasu, Labeo rohita, Leptobarbus hoeveni, Megalobrama amblycephala, Mylopharyngodon piceus, Notemigonus crysoleucas, Osteochilus hasselti, Parabramis pekinensis, Puntius gonionotus, Puntius javanicus, Rutilus rutilus, Tinca tinca, Misgurnus anguillicaudatus, Ictiobus cyprinellus, Ichthyoelephas humeralis, Prochilodus reticulatus, Brycon moorei, Colossoma macropomum, Piaractus brachypomus, Piaractus mesopotamicus, Ictalurus melas, Ictalurus punctatus, Chrysichthys nigrodigitatus, Siluris glanis, Pangasius pangasius, Pangasius sutchi, Clarias anguillaris, Clarias batrachus, Clarias fuscus, Clarias gariepinus, Clarias macrocephalus, Heterobranchus bidorsalis, Heterobranchus longifilis, Rhamdia sapo, Hoplosternum littorale, Esox lucius, Plecoglossus altivelis, Coregonus albula, Coregonus lavaretus, Oncorhynchus gorbuscha, Oncorhynchus keta, Oncorhynchus kisutch, Oncorhynchus masou, Oncorhynchus mykiss, Oncorhynchus nerka, Oncorhynchus tshawytscha, Salmo salar, Salmo trutta, Salvelinus alpinus, Salvelinus fontinalis, Salvelinus namaycush, Gadus morhua, Odontesthes bonariensis, Monopterus albus, Centropomus undecimalis, Lates calcarifer, Lates niloticus, Maccullochella peeli, Macquaria ambigua, Morone saxatilis, Dicentrarchus labrax, Epinephelus akaara, Epinephelus areolatus, Epinephelus tauvina, Plectropomus maculatus, Bidyanus bidyanus, Micropterus salmoides, Perca fluviatilis, Stizostedion lucioperca, Pomatomus saltatrix, Seriola dumerili, Seriola quinqueradiata, Trachinotus blochii, Trachinotus carolinus, Trachinotus goodei, Trachurus japonicus, Lutjanus argentimaculatus, Ocyurus chrysurus, Acanthopagrus schlegeli, Diplodus sargus, Evynnis japonica, Pagrus major, Pagrus pagrus, Rhabdosargus sarba, Sparus aurata, Sciaenops ocellatus, Aequidens rivulatus, Cichlasoma maculicauda, Cichlasoma managuense, Cichlasoma urophthalmus, Etroplus suratensis, Oreochromis andersonii, Oreochromis aureus, Oreochromis macrochir, Oreochromis mossambicus, Oreochromis niloticus, Oreochromis spilurus, Oreochromis urolepis, Sarotherodon melanotheron, Tilapia guineensis, Tilapia rendalli, Tilapia zillii, Liza aurata, Liza macrolepis, Liza parsia, Liza ramada, Liza saliens, Liza tade, Mugil cephalus, Mugil curema, Mugil liza, Dormitator latifrons, Oxyeleotris marmorata, Siganus canaliculatus, Siganus guttatus, Siganus rivulatus, Thunnus maccoyii, Thunnus thynnus, Anabas testudineus, Trichogaster pectoralis, Helostoma temmincki, Osphronemus goramy, Channa argus, Channa micropeltes, Channa punctatus, Channa striata, Psetta maxima, Paralichthys olivaceus* and Solea *vulgaris.* In other preferred embodiments of the invention, the aquatic species is selected from the group consisting of crustaceans, in particular from the group of crustacean species belonging to one of the following families: Penaeidae, Sergestidae, Palaemonidae, Nephropidae, Astacidae, Cambaridae, Parastacidae, Palinuridae, Portunidae and Potamidae, such as from the group of crustacean species consisting of *Metapenaeus dobsoni, Metapenaeus endeavouri, Metapenaeus ensis, Metapenaeus monoceros, Penaeus aztecus, Penaeus chinensis, Penaeus esculentus, Penaeus indicus, Penaeus japonicus, Penaeus kerathurus, Penaeus merguiensis, Penaeus monodon, Penaeus notialis, Penaeus paulensis, Penaeus penicillatus, Penaeus schmitti, Penaeus semisulcatus, Penaeus setiferus, Penaeus stylirostris, Penaeus subtilis, Penaeus vannamei, Xiphopenaeus kroyeri, Acetes japonicas, Macrobrachium malcolmsonii, Macrobrachium rosenbergii, Palaemon serratus, Homarus americanus, Homarus gammarus, Astacus astacus, Astacus leptodactylus, Pacifastacus leniusculus, Procambarus clarkii, Cherax destructor, Cherax quadricarinatus, Cherax tenuimanus, Panulirus longipes, Portunus trituberculatus, Scylla serrata* and *Eriocheir sinensis.* In other preferred embodiments of the invention, the aquatic species is selected from the group consisting of mollusks, in particular from the group of mollusk species belonging to one of the following families: Haliotidae, Littorinidae, Strombidae, Mytilidae, Arcidae, Pteriidae, Hiatellidae, Pectinidae, Ostreidae, Cardiidae, Tridacnidae, Mactridae, Solecurtidae, Corbiculidae, Veneridae, Myidae and Hiatellidae, such as from the group of mollusc species consisting of *Haliotis discus, Haliotis diversicolor, Haliotis midae, Haliotis rufescens, Haliotis tuberculata, Littorina littorea, Strombus gigas, Aulacomya ater, Choromytilus chorus, Mytilus californianus, Mytilus chilensis, Mytilus coruscus, Mytilus edulis, Mytilus galloprovincialis, Mytilus planulatus, Perna canaliculus, Perna indica, Perna perna, Perna viridis, Anadara granosa, Scapharca broughtonii, Scapharca subcrenata, Pinctada fucata, Pinctada margaritifera, Pinctada maxima, Pteria penguin, Aequipecten opercularis, Argopecten irradians, Argopecten purpuratus, Argopecten ventricosus, Chlamys farreri, Chlamys islandica, Chlamys nobilis, Patinopecten yessoensis, Pecten fumatus, Pecten maximus, Pecten novaezelandiae, Placopecten magellanicus, Crassostrea belcheri, Crassostrea corteziensis, Crassostrea gigas, Crassostrea iredalei, Crassostrea madrasensis, Crassostrea rhizophorae, Crassostrea rivularis, Crassostrea virginica, Ostrea chilensis, Ostrea edulis, Ostrea lurida, Saccostrea commercialis, Saccostrea cuccullata, Saccostrea echinata, Cerastoderma edule, Tridacna derasa, Tridacna gigas, Mactra glabrata, Mactra veneriformis, Spisula solidissima, Sinonovacula constricta, Corbicula fluminea, Corbicula japonica, Mercenaria mercenaria, Meretrix lusoria, Meretrix meretrix, Paphia undulata, Protothaca staminea, Ruditapes decussatus, Ruditapes philippinarum, Saxidomus giganteus, Venerupis pullastra, Mya arenaria* and *Panopea abrupta.*

In particularly preferred embodiments of the invention, the aquatic species is selected from the group consisting of shrimp or prawn (Penaeidae), in particular tiger shrimp (especially *Penaeus monodon*) and whiteleg shrimp (*Penaeus vannamei*); Tilapia, Rohu, Catfish and Pangas. In certain preferred embodiments of the invention, the aquatic species is not rainbow trout (*Oncorhynchus mykiss*), more preferably the aquatic species is not a species from the genus *Oncorhynchus,* more preferably it is not a species from the Family of Salmonidae.

As already stated herein before, the uses of the invention result in and/or are aimed at the attainment of one or more beneficial effects in relation to productivity, as defined in the claims. In the uses of the invention, as defined herein, the composition comprising the bioavailable silicic acid compound may be regarded a fertilizer, feed additive and/or biostimulant. These terms and their meaning, in the context of the present invention, are well known and understood by those skilled in the art.

In one embodiment of the invention, the uses result in and/or are aimed at enhancing growth of the aquatic animal. In one embodiment of the invention, the uses result in and/or are aimed at increasing the weight of the aquatic animal. In particularly preferred embodiments of the invention, enhanced growth is reflected by an increase in average weight of the aquatic animals of at least 5 %, compared to average weight of the aquatic animals attained under the same conditions but without the silicic acid treatment. In particularly preferred embodiment said increase in average weight is at least 6 %, at least 7 %, at least 8 %, at least 9 %, at least 10 %, at least 11 %, at least 12 %, at least 13 %, at least 14 % or at least 15 %.

In one embodiment of the invention, the uses result in and/or are aimed at accelerating growth of the aquatic animal. In one embodiment of the invention the uses result in and/or are aimed at reducing the time to harvest. In particularly preferred embodiments of the invention, accelerated growth is reflected by a reduction in the average time between hatching and harvesting, compared to the average time required under the same conditions, but without silicic acid treatment. In particularly preferred embodiment said reduction is at least 5 days, preferably at least 7 days, at least 8 days, at least 9 days, at least 10 days, at least 11 days, at least 12 days, at least 13 days, at least 14 days or at least 15 days.

Also disclosed herein are uses resulting in and/or are aimed at increasing the overall yield. In particular, yield may be increased by at least 5 %, typically on a weight basis, compared to yield attained under the same conditions but without the silicic acid treatment. More iIn particular, said yield may be increased by at least 10 %, at least 15 %, at least 20 %, at least 25 %, at least 30 %, at least 35 % or at least 40 %.

In one embodiment of the invention, the methods and uses result in and/or are aimed at improving feed utilization. In one embodiment of the invention, the methods and uses result in and/or are aimed at improving the feed conversion rate, referred in the art as FCR, which is a measure of an animal's efficiency in converting feed mass into increases of the desired output. For food-producing animals, the output is the mass gained by the animal. Specifically, unless otherwise explicitly stated in the disclosure, FCR is calculated as feed intake divided by weight gain, all over a specified period. Improvement in FCR means reduction of the FCR value. A FCR improvement of 5 % means that the FCR was reduced by 5 %, compared to the FCR attained under the same conditions but without the silicic acid treatment. In particularly preferred embodiment, improvements in feed utilization are reflected by an FCR decrease of at least 2.5 %, preferably at least 5 %, at least 7.5 %, at least 10 %, at least 12.5 %, or at least 15 %.

Also disclosed herein are uses resulting in and/or are aimed at improving the water quality.

Also disclosed herein are uses resulting in and/or are aimed at increasing dissolved oxygen level and/or maintaining appropriate dissolved oxygen levels. In particular, the dissolved oxygen level may be increased by at least 0.5 mg/L, at least 0.75 mg/L, at least 1 mg/L, at least 1.25 mg/L, or at least 1.5 mg/L, compared to dissolved oxygen levels attained under the same conditions but without the silicic acid treatment. More in particular, the dissolved oxygen level may be increased to a level or maintained at a level within the range of 3-20 mg/L, preferably within the range of 4-17.5 mg/L, most preferably within the range of 5-15 mg/L. As mentioned herein before, the use of fertilizers can result in algae bloom and eventually decrease dissolved oxygen levels. Hence, the use of compositions comprising bioavailable silicic acid compounds as a fertilizer, without a decreasing effect on dissolved oxygen levels is an advantage over the use of certain other fertilizers.

Also disclosed herein are uses resulting in and/or are aimed at decrease of the concentration of ammonia. In particular, the ammonia level may be decreased by at least 0.005 mg/L, at least 0.01 mg/L, at least 0.05 mg/L, at least 0.1 mg/L or at least 0.5 mg/L compared to ammonia levels attained under the same conditions but without the silicic acid treatment. More in another particular, the ammonia level may be decreased to and/or maintained at a level within the range of 0.015-0.15 mg/L, preferably within the range of 0.05-0.1 mg/L.

Also disclosed herein are uses resulting in and/or are aimed at improving the pH of the water and/or maintaining the pH at appropriate levels. In particular, the pH may be increased by at least 0.25 pH units, compared to pH levels attained under the same conditions but without the silicic acid treatment. More in particular, said pH increase may be at least 0.5 pH units, at least 0.75 pH units, at least 1 pH unit, at least 1.25 pH unit, or at least 1.5 pH units. The pH level may be increased to a level or maintained at a level within the range of 6-10, preferably within the range of 6.5-9.

Also disclosed herein are uses resulting in and/or are aimed at decreasing salinity and/or preventing salinization and/or hypersalinization of the water.

Also disclosed herein are uses resulting in and/or are aimed at enhancing growth of phytoplankton, especially the diatoms in the water. In particular, the phytoplankton content of the water, especially the diatoms content of the water, may be increased by at least 5 %, on a wt/wt basis, compared to the phytoplankton (or diatoms) content attained under the same conditions but without the silicic acid treatment. More in particular, said content may be increased by at least 6 %, at least 7 %, at least 8 %, at least 9 %, at least 10 %, at least 11 %, at least 12 %, at least 13 %, at least 14 % or at least 15 %.

Also disclosed herein are uses resulting in and/or are aimed at reducing the ecological impact of aquaculture.

In particularly preferred embodiments of the invention, the uses do not result in and/or are not aimed at any effects on the health of the aquatic animals, such as the curing of a pathology or health condition, preventing a pathology or health condition and/or alleviating one or more symptoms of a pathology or health condition. In particularly preferred embodiments of the invention, the uses are non-therapeutic, e.g. non-curative and non-prophylactic.

In a particularly preferred embodiment of the invention the uses do not affect and/or are not aimed at affecting the immune system, such as strengthening the immune system. In a particularly preferred embodiment of the invention the uses do not result in and/or are not aimed at prevention and/or treating of *Saprolegnia* infection, more preferably the uses of the invention do not result in and/or are not aimed at prevention and/or treating fungal infection or microbial infection in aquatic animals, in particular in rainbow trout or a species belonging to the Salmonidae family.

Unless otherwise defined, all terms used in disclosing the invention, including technical and scientific terms, have the meaning as commonly understood by one of ordinary skill in the art to which this invention belongs.

"A", "an", and "the" as used herein refer to both singular and plural forms unless the context clearly dictates otherwise. By way of example, "a compartment" refers to one or more than one compartment.

"About" as used herein referring to a measurable value such as a parameter, an amount, a temporal duration, and the like, is meant to encompass variations of +/-10% or less, more preferably +/-5% or less, even more preferably +/-1% or less of and from the specified value, in so far such variations are appropriate to perform in the disclosed invention. However, it is to be understood that the value to which the modifier "about" refers is itself also specifically disclosed.

"Comprise", "comprising", "comprises" and "comprised of" as used herein are synonymous with "include", "including", "includes" or "contain", "containing", "contains" and are inclusive or open-ended terms that specify the presence of what follows, e.g. a component, and do not exclude or preclude the presence of additional, non-recited components, features, element, members, steps, known in the art or disclosed therein.

The recitation of numerical ranges by endpoints includes all numbers and fractions subsumed within that range, as well as the recited endpoints. The skilled person will appreciate that the present invention can incorporate any number of the specific features described above.

Throughout this text, the use of terms in brackets, usually means that the term within brackets specifies a possible option or a possible meaning and should thus not be considered limiting.

Advantages of the invention will become apparent from the following examples, which are given below as mere illustrations, and are non-limitative.

### Description of the Figure

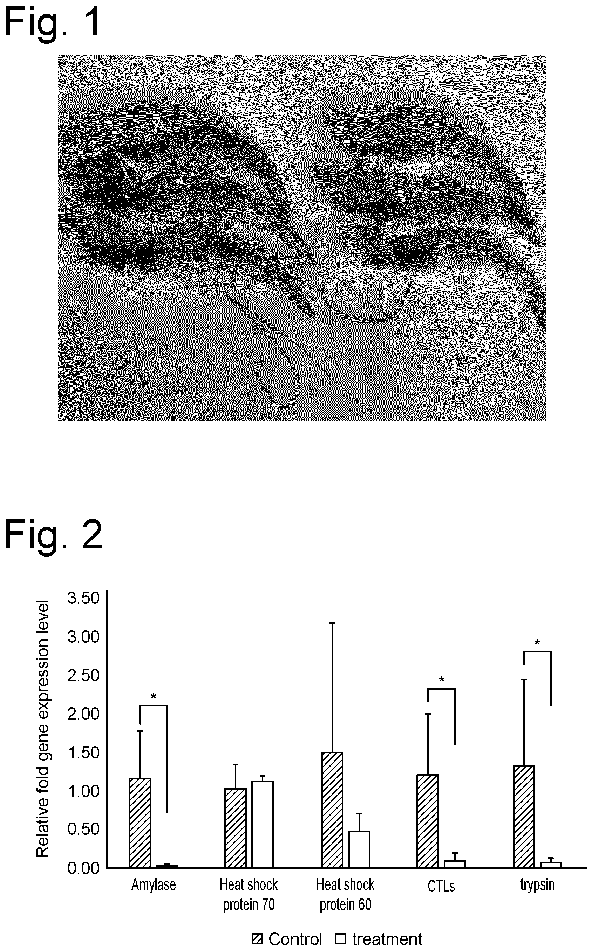

Figure 1: Image of shrimp Litopenaeus vannamei sampled on termination of a growth trial (8-weeks) evaluation. Shrimp from the control group, depicted on the righthand sight, have an average weight of 9.48g. Shrimp from the treatment group, depicted on the left-hand sight, attained a 12.49g average weight.
Figure 2. Gene expression relative to 'housekeeping' gene showing up-regulation and down- regulation of specific genes concerned with growth response in shrimp comparing control group with silicic acid treated shrimp after 56 days. Relative fold gene expression level of growth-related genes of control and treatment group. Statistical analysis was done using t-test: paired two sample for mean (p > 0.05), compared to control.

### Experimental

### Experiment 1: Trials on shrimps (India) I

Trials with bioavailable silicic acid compounds, with or without other elements (like boron, molybdenum and zinc), were performed to evaluate the growth of whiteleg shrimps (Litopenaeus vannamei) in brackish ponds in Gujarat (India) with sizes between 0,6 and 1 hectare. Water quality parameters, including salinity, dissolved oxygen, pH and total ammonia nitrogen (TAN) were monitored regularly in both control and treated ponds. The growth rate of the shrimps was monitored regularly. Total number was counted and mean body weight of shrimp was measured. Based on recording the weight of shrimp and counting the number of shrimps, weight gain, feed conversion ratio (FCR) and survival were calculated.

In 2017, the first experiments in Gujarat (India) showed an average weight gain of the shrimps of +26% compared to control pond. In a second trial an increase of +17,5% (compared to control) was found.

Other trial results:
1. Zooplankton and Phytoplankton levels were increased substantially in the treated ponds
2. Dissolved oxygen rate increased marginally
3. PH values in the treated ponds were more favorable in the treated ponds. Fish can become stressed in water with a pH < 6.5 and > 9. Under these conditions fish / shrimp growth is limited and reproduction will cease. So, optimizing pH appeared to be an important issue as well.
4. The concentration of N (ammonia) decreased significantly compared to control.
5. The economics of using bioavailable silicic acid compounds show a cost-benefit ratio of 1 : 3 - 4.

### Experiment 2: Trials on shrimps (India) II

From 2018 - 2019 experiments similar to experiment have been done in Gujarat-India on whiteleg shrimps to confirm the earlier (positive) data.

In this experiment on 4 ponds, with a size between 0,6 - 1 hectare, every 3 days bioavailable silicic acid compound was applied (broadcasting) to the ponds with brackish water being circulated in the ponds.

It was shown that the average yield increase of shrimps is +24,27% in the treated ponds. The feed conversion rate (FCR) showed a more economic use in all 4 ponds being an average of 1,5 for control ponds to an average of 1,37 for the treated ponds.

The water quality parameters, like salinity, pH, dissolved oxygen and total ammonia were improved.

### Experiment 3: Trials on shrimps (India) III

In 2000 further experiments were done in Gujarat-India on whiteleg shrimps. In these trials the efficacy of the application (broadcasting) of higher doses of bioavailable silicic acid compounds, every 15 days in 8 ponds (with controls), with a seize between 0,6 - 0.8 hectare, filled with brackish water being circulated in the ponds. Every 15 days 1.5 liters of a 2.5 % product (bioavailable silicic acid compound in aqueous medium) was added to each of the ponds (except controls) containing an estimated 10.000 m³ of water (0.6-0.8 hectare, with an average depth of 1.2 meters).

It was shown that the average yield increase of shrimps is +18,3 % in the treated ponds (compared to control).

The food conversion rate (FCR) showed a more economic use in all 8 treatment ponds, with an average of 1,52 for control ponds and an average of 1,45 for the treated ponds.

The water quality parameters, like salinity, pH, dissolved oxygen improved marginally, while the total ammonia levels improved (decreased) significantly.

### Experiment 4: Trials on shrimps (India) IV

In 2020 experiments were done in Gujarat-India on whiteleg shrimps to evaluate the efficacy of daily application of silicic acid (compared to experiment 3) to the shrimp being applied once or twice a day to 6 ponds (with controls) with a size between 0,6 - 1 hectare, filled with brackish water being circulated in the ponds. The results were similar: overall increase of the yield: +19%.

### Experiment 5: Trials on Whiteleg Shrimps in Vietnam

Trials are done in Northern part, Central part and Southern part in Vietnam, 2020-2021. Similar results were obtained from all testing locations:
1. Improvement of water quality (based on dissolved oxygen, pH, NH3, alkalinity and salinity);
2. Improvement of all growth parameters: larger shrimps (average: + 14%) and higher yields (average of: + 18.7%);

The conclusions of the researchers were:
Central Vietnam: Bioavailable silicic acid compound influenced both growth and yield of whiteleg shrimp. Experiments gave remarkably good results for environment, growth, survival, and energy efficiency of shrimp production. Moreover, the color of the water of treated ponds was much better than the control ponds, presumably due to the enhancement of phytoplankton, algae, and diatoms in the pond.

Northern Vietnam: The use of Silicic Acid has improved the water environment. The data indicates that the treatment created a more favorable condition for shrimp growth.

### Experiment 6: Trial on shrimp in Bangladesh

Tiger shrimps (Penaeus monodon), also named giant tiger prawn, asian tiger prawn or black tiger shrimp, is one of the most popular cultured shrimp species in the world.

A small scale trial was carried out in 2019 in Chittagong-Bangladesh in three ponds of + 2,5 ha. The water volume of the farms was routinely changed every 5 days and the silicic acid was applied after each change of water. The overall yield increase (compared to control) was 18,4%.

### Experiment 7: Trials on fish in Bangladesh

Bangladesh is considered one of the most suitable countries in the world for freshwater aquaculture, because of its favorable agro-climatic conditions. To establish the maximum production potential of sweet water and brackish water based aquaculture using bioavailable silicic acid compounds on mono and mixed culture of five selected fish species, experiments were conducted for a period of 24 months in Bangladesh.

In 2018 experiments on several types of (sweet water) fish: Tilapia, Rohu and other carp- species, Catfish and Pangas (Pangasius hypophthalmus) were done in Bangladesh with soluble (subcolloidal) silicic acid.

### A. Tilapia.

In Mymensingh district (Bangladesh) most farmers (82%) practice small-scale tilapia farming with carps and catfish or tilapia as a monoculture under semi-intensive or intensive systems. Farmers stock their ponds from as early as April to May and harvest tilapia after four months and repeat this a second time, usually two crops per year. The most common supplementary feed for small-scale tilapia farming is a mixture of rice bran, wheat bran and mustard oil cake. Compositions comprising bioavailable silicic acid compounds (2.5 %) in combination with boric acid (0.2 %), zinc (1.5 %) and manganese (0.3 %), stabilized with PEG, were applied to 4 different ponds (with controls) every 5 days (0.5 liter of the composition added to each pond). The main observation, based on 2 consecutive breeding periods was a faster growth of tilapia. The average yield was 5600 kg/ha compared to 4300 kg/ha, equating to an (overall) increase of yield of + 30%.

### B. Rohu (Labeo rohita) and two other major carp species.

Rohu (Labeo rohita) is the most important among the three Indian major carp species used in carp polyculture systems in Bangladesh. The nursery-raised fry of 20-25 mm are further reared for two-three months to 80-100 mm (6-10 g) fingerlings in ponds of 0.2 ha. Here, rohu are grown together with other carp species at combined densities of 0.2-0.3 million fry/ha, with the rohu constituting about 30-40 percent of the total. Pond fertilization was done with both organic and inorganic fertilizers, and supplementary feeding with the conventional mixture of rice bran and oil cake. Bioavailable silicic acid compound (composition comprising 2.5 % silicic acid, PEG, 0.6 % boric acid and 0.1 % molybdenum) was applied to 9 ponds (with controls) with 5 days interval (0.5 liter of the composition was added to each pond every time). Based on the overall results of the 9 ponds the overall yield increased from 2,3 tonnes/ha/year to 3,1 tonnes, an increase of 35%.

### C. Pangasius hypophthalmus

Pangas belonging to the genus of medium-large catfishes, is a relatively new and fast-growing fish species that has great potential for production and export growth in Bangladesh. Polyculture of pangasius (P. hypophthalmus) with carps is the existing culture practice along with monoculture being practiced by many households in Bangladesh.

Trials have been done in 2019 in Mymensingh region on 4 ponds with an average of 1,4 ha and an average depth of 1,5 m in the dry season and 2 m. in the rainy season (May to November). For feeding standard commercial pelleted feeds were used. Bioavailable silicic acid compound was applied to 4 ponds and the combination of soluble silicic acid, boric acid and zinc was applied to another 4 ponds (all with controls) with 5 days interval for application (0.5 liters of the composition added to each pond every time). Based on the overall results of the 8 ponds the overall yield increased from 11.400 kg/ha/year to 13.300 kg/ha/year being an increase of 16,6%. Next, it was shown that the water quality improved: pH decreased from 8,2 (at the beginning of the trial) to 7,9 while the total ammonia decreased during the trial and at the end of the trial the total ammonia was (on average) 1.1 ppm in controls to 0,35 ppm in the treated ponds.

The conclusion of the researcher: 'to improve the growth of pangasius, is is important to upgrade the existing pangasius management practices with the silicic acid technology'.

### Conclusion

During the period of 2017-2021 research has been done on several kinds of shrimps and fishes in India, Bangladesh and Vietnam. Although further trials are ongoing, it is already clear that bioavailable silicic acid compounds are capable to increase the growth of shrimps and fishes and to stimulate the growth of beneficial (levels of) algae, diatoms and many types of water plants.

Trials have been done with bioavailable silicic acid compounds alone or in combinations with Boron and, in some cases, Copper, Molybdenum, Zinc and others. All combinations have been shown very effective in increasing the growth of fish and shrimp, the growth of algae and diatoms and improving the water quality based on the parameters pH, dissolved oxygen, total ammonia and alkalinity.

### Experiment 8 - Expression of genes for nutrient assimilation in Pacific Whiteleq shrimp (L. Vannamei) treated with bioavailable silicic acid in rearing ponds

In rapidly developing shrimp, many genes are also associated with nutrient assimilation and growth such as anabolic metabolism. Such genes as trypsin and amylase are involved in digestion and will be either upregulated or down regulated depending on need. As a follow-on from previous trials the effects of a bioavailable silicic acid product in accordance with the invention on expression of these genes was determined for *L. vannamei.*

### Materials and methods

An 8-week growth trial study on Pacific Whiteleg shrimp (*Litopenaeus vannamei*) (1.0 g/shrimp) in duplicate was conducted in a 3,200 m2 earthen pond (100,000 shrimps per 1,600 m2) at the GAP certified private shrimp farm, Nakhon Pathom province, Thailand. Nakhon Pathom is one of the central provinces of Thailand. The pond size was 2 Rai (1 Rai is 1,600 m²). Stocking density is 100,000 shrimps / 1 Rai.

Shrimp were fed by automatic feeder four times a day according to the farming standard. The feed was obtained from the Phoka Feed Mill Co., Ltd., Thailand (284/1 Moo 1 Mueang Nakhon Pathom, Nakhon Pathom, 73000 Thailand). The bespoke shrimp feed specification was Crude Protein 38%, Crude Fat 7%, and diet pellet sizes of 1.5 mm; 2.0 mm; 2.5 mm that were assigned across the trial period to optimise maximum intake.

All shrimp responded well to the respective diet and conditions over the 56-day period. At the end of the grow-out period, shrimp were sampled for the growth and feed utilisation metrics. Water quality parameters were measured three times daily. At the end of the trial, shrimps were assessed for the growth performance as described below. Shrimp from each treatment group were examined and the hepato-pancreas removed carefully from 10 individual animals and processed as described below.

For RNA extraction, hepatopancreases were extracted by using trizol reagent (Invitrogen, USA). Briefly, 1 ml of trizol reagent were added into 50 - 100 mg of tissue samples. then, the samples were homogenized at 3,500 rpm for 1 min by using Micro Smash MS-100R (TOMY, Japan). After incubated sample at RT for 5 min, 200 µl of chloroform were added and mixed vigorous. The samples were centrifuged at 12,000 rpm at 4°C for 15 min. after collected supernatant into new 1.5 ml tube, 1 volume of isopropanol were added and incubated at -20°C for 2 h or O/N. Next, RNA pellets were collected by centrifuged at 12,000 rpm at 4°C for 15 min. then, the pellets were washed with 500 µl of 75% ethanol and dried pellet at 65 °C. Lastly, 30 µl of RNase free water was added for dissolved RNA pellet. RNA concentration was measured by using nanodrop spectrophotometer (Thermo Fisher Scientific, USA).

After RNA extraction, the DNA were eliminated by using DNase I (Thermo Fisher Scientific, USA). The reaction for DNase I treatment consisted of 1 µl of 10X buffer, 1 µl of DNase I and 1 - 2 µg of RNA. Then, the volume was adjusted to 10 µl by using RNase free water. After preparing the reaction, the mixtures were incubated at 37 °C for 30 min and subsequently added 1 µl of EDTA and incubated at 65°C for 10 min to stop the reaction.

cDNA in this experiment was prepared by using Viva 2-step RT-PCR kit (Vivantis, Malaysia). Firstly, 1 ug of DNase treated RNA, 1 µl of oligo dT primer and 1 µl of 10 mM dNTP were added into 0.2 ml tube and incubated at 65 °C for 5 min. After being quick chilled on ice, 2 µl of 10X buffer and 100 U of M-MuLV reverse transcriptase were added and adjusted volume to 20 µl with RNase free water. Next, the mixtures were incubated at 42 °C for 90 min and then incubated at 85 °C for 5 min for stop reaction.

Real-time PCR was used to detected growth genes in shrimp. The primers used were as displayed by Table 1

**Table 1. List of genes selected for real-time PCR determination**

| group | Genes |
|---|---|
| Digestive function and stress tolerance genes | α - amylase |
| | Heat shock protein 60 |
| | Heat shock protein 70 |
| | CTL (C-type lectin) |
| | Trypsin |
| Reference gene | B-actin (housekeeping gene) |

Reaction mixture for real-time PCR was composed of 1 µl of 100 cDNA, 5 µl of 2x qPCRBIO SyGreen mix, forward and reverse primer at final concentration 0.2 µM and adjusted volume up to 10 µl by nuclease free water. The real-time PCR condition was initially from a pro-denaturation at 95°C for 5 min, then subsequently a denaturation phase at 95°C for 30 sec, annealing at 58°C for 30 seconds. This was followed by 40 repeated cycles and final extension at 72°C for 5 min. The relative fold gene expression level was calculated by using the formula below:
Relative fold gene expression level = 2^{-ΔΔCT}
CT = Cycle number of samples
ΔCT = CT (gene of interest) - CT (housekeeping gene)
ΔΔCT = ΔCT (treated sample) - ΔCT (untreated sample)

### Results & Discussion

Shrimp performed significantly better when exposed to available silicic acid for 8 weeks under the pond rearing conditions. The results of the growth and feed performance of shrimp subjected to the experimental treatments are displayed in Table 2. These show a very significant elevation in total biomass and mean body weight of animals at the end of the trial period (mean weight of control 9.48, and 12.49 for the silicic acid product) as displayed in Figure 1. The SGR (Specific Growth Rate) defined as the mean daily live weight gain (% day ⁻¹) also reflects the much higher and significant effect of silicic acid addition to rearing ponds. Protein Efficiency Ratio (PER) is a measurement of gain of biomass per unit of protein intake and reflects the conversion efficiency of dietary protein conversion in gross terms. It is evident that from Table 1 there is a marked increase in PER showing that silicic acid has enhanced the protein conversion from 1.42 to 1.76.

The principle aim of this study was to ascertain the effects of silicic acid on gene expression and link to performance. Therefore, due to the known importance of regulatory genes in the control of protein and energy assimilation, the various genes were selected in accordance with their importance to shrimp production traits as shown in Table 2.

**Table 2. Growth performance of cultured Pacific Whiteleg shrimp at two different concentrations of silicic acid**

| **Growth performances** | **Without silicic acid** | **With silicic acid** | ***P*-value** |
|---|---|---|---|
| Total biomass (kg of harvested shrimp) | 710 ± 6.24 **^{B}** | 1,047 ± 208.25 ^{A} | 0.024 |
| Final weight (g/shrimp) | 9.48 + 0.49 | 12.49 ± 2.46 | 0.053 |
| Feed conversion ratio | 1.46 ± 0.02^{B} | 1.18 ± 0.05 ^{A} | 0.001 |
| Specific growth rate (%/day) | 2.94 + 0.05 ^{B} | 3.21 ± 0.20 ^{A} | 0.044 |
| Average daily gain (g/day) | 0.09 ± 0.00 | 0.12 ± 0.02 | 0.053 |
| Survival rate (%) | 62.6 ± 3.8 ^{B} | 69.8 ± 4.5 ^{A} | 0.049 |
| Protein efficiency ratio | 1.42 ± 0.02 ^{B} | 1.76 ± 0.08 ^{A} | 0.001 |
| Apparent net protein utilization (%) | 27.2 + 0.1 ^{B} | 36.4 ± 1.4^{A} | 0.000 |

| | | | |
|---|---|---|---|
| Different superscripts in the same row means significantly different (P < 0.05) | | | |

To compare differences between control and treatment shrimp, real-time PCR was performed by using 6 genes including α - amylase, Heat shock protein 60, Heat shock protein 70, Chitin, cathepsin-I candidates' gene for feed efficiency (CTLs) and trypsin. The results demonstrated that in case of α - amylase, CTLs and trypsin gene expressions, there appeared to be significant differences between the control and treatment group (Figure 2). The down regulation of the two specific genes coding for the production of amylase (starch degradation) and trypsin (protein degradations) may imply a sparing action due to the enhanced effect of silicic acid on primary production of planktonic organisms in ponds. Bioavailable silicic acid has proven a key nutrient for diatoms and plant life and indirectly zooplankton providing natural exogenous digestive enzymes for shrimp reducing the need for biosynthesis and energy demand. Such assisted digestion will require much less endogenous intestinal enzyme production leading to efficient gains in feed utilisation and efficiency.

### Conclusion

In conclusion, the effect of adding a bioavailable silicic acid product according to the invention has pronounced effects on specific genes that influence shrimp performance in relation to digestive functional efficiency.

## Claims

1. Use of a composition comprising a bioavailable silicic acid compound in the farming of aquatic animals selected from the group consisting of fish, crustaceans and mollusks, wherein the use has the objective of and/or results in one or more of:
- enhancing growth of the aquatic animal;
- accelerating growth of the aquatic animal;
- increasing the weight of the aquatic animal;
- reducing the time to harvest;
- improving feed utilization; and
- increasing the feed conversion rate.

2. Use according to claim 1, wherein the aquatic species is a species from the family of *Salmonidae.*

3. Use according to claim 1, wherein the aquatic species is selected from the group consisting of crustaceans and mollusks.

4. Use according to any one of the preceding claims, wherein at least 90 mol.% of the silicon contained in the composition is in the form of a bioavailable silicic acid compound.

5. Use according to any one of the preceding claims, wherein the bioavailable silicic acid compound is selected from the group consisting of monomeric silicic acid, dimeric silicic acid, oligomeric silicic acid and polymeric silicic acid in subcolloidal form and combinations thereof.

6. Use according to claim 5, wherein the bioavailable silicic acid compound is in the form of subcolloidal particles having a size in the range of 1-10 nm, as determined by ²⁹Si NMR spectroscopy.

7. Use according to claim 6, wherein at least 50 % of the silicic acid containing particles has a size within the range of 1-10 nm.

8. Use according to any one of the preceding claims, wherein the composition comprises an acidified aqueous solution or dispersion of bioavailable silicic acid compounds in the form of subcolloidal particles, preferably in combination with boric acid and/or a water absorbing additive.

9. Use according to any one of the preceding claims, wherein the use has the objective of and/or results in an increase in average weight of the aquatic animals of at least 5 %, compared to average weight of the aquatic animals attained under the same conditions but without the silicic acid treatment.

10. Use according to any one of claims 1 and 3-9, in the farming of a species selected from the group of Shrimps.

11. Use according to any one of claims 1 and 4-9, in the farming of a species selected from the group of fishes, preferably selected from the group of *cichlidae, cyprinidae* and *siluridae,* more preferably selected from the group of Tilapia, Rohu, Catfish and Pangas.

12. Use according to any one of the preceding claims, wherein the use comprises the addition of the composition comprising bioavailable silicic acid compounds to the water in which the aquatic animals are kept.

13. Use according to any one of the preceding claims, wherein the use comprises the steps of blending the composition comprising bioavailable silicic acid compounds with feed and adding the feed to the water in which the aquatic animals are kept.

## Patentansprüche

1. Verwendung einer Zusammensetzung, die eine bioverfügbare Kieselsäureverbindung umfasst, in der Zucht von Wassertieren, ausgewählt aus der Gruppe bestehend aus Fischen, Schalentieren und Weichtieren, wobei die Verwendung eines oder mehrere der folgenden Ziele verfolgt und/oder zu einem oder mehreren der folgenden Ergebnisse führt:
- Fördern des Wachstums des Wassertieres;
- Beschleunigen des Wachstums des Wassertieres;
- Erhöhen des Gewichts des Wassertieres;
- Verkürzen der Zeit bis zur Entnahme;
- Verbessern der Futterverwertung; und
- Erhöhen der Futterumwandlungsrate.

2. Verwendung nach Anspruch 1, wobei die aquatische Spezies eine Spezies aus der Familie der *Salmonidae* ist.

3. Verwendung nach Anspruch 1, wobei die aquatische Spezies aus der Gruppe ausgewählt ist, die aus Schalentieren und Weichtieren besteht.

4. Verwendung nach einem der voranstehenden Ansprüche, wobei mindestens 90 Mol-% des in der Zusammensetzung enthaltenen Siliciums in Form einer bioverfügbaren Kieselsäureverbindung vorliegen.

5. Verwendung nach einem der voranstehenden Ansprüche, wobei die bioverfügbare Kieselsäureverbindung aus der Gruppe ausgewählt ist, die aus monomerer Kieselsäure, dimerer Kieselsäure, oligomerer Kieselsäure und polymerer Kieselsäure in subkolloidaler Form und Kombinationen dieser besteht.

6. Verwendung nach Anspruch 5, wobei die bioverfügbare Kieselsäureverbindung in Form von subkolloidalen Partikeln mit einer Größe im Bereich von 1-10 nm vorliegt, wie durch ²⁹Si-NMR-Spektroskopie bestimmt.

7. Verwendung nach Anspruch 6, wobei mindestens 50 % der kieselsäurehaltigen Partikel eine Größe im Bereich von 1-10 nm aufweisen.

8. Verwendung nach einem der voranstehenden Ansprüche, wobei die Zusammensetzung eine angesäuerte wässrige Lösung oder Dispersion von bioverfügbaren Kieselsäureverbindungen in Form von subkolloidalen Partikeln, vorzugsweise in Kombination mit Borsäure und/oder einem wasserabsorbierenden Additiv, umfasst.

9. Verwendung nach einem der voranstehenden Ansprüche, wobei die Verwendung das Ziel einer Erhöhung des Durchschnittsgewichts der Wassertiere um mindestens 5 % im Vergleich zu dem Durchschnittsgewicht der Wassertiere, das unter den gleichen Bedingungen, jedoch ohne die Kieselsäurebehandlung, erreicht wurde, verfolgt oder dazu führt.

10. Verwendung nach einem der Ansprüche 1 und 3-9, in der Zucht einer Spezies, ausgewählt aus der Gruppe der Garnelen.

11. Verwendung nach einem der Ansprüche 1 und 4-9 in der Zucht einer Spezies, ausgewählt aus der Gruppe der Fische, vorzugsweise ausgewählt aus der Gruppe der *Cichlidae, Cyprinidae* und *Siluridae,* noch bevorzugter ausgewählt aus der Gruppe der Tilapia, Rohu, Welse und Pangasius.

12. Verwendung nach einem der voranstehenden Ansprüche, wobei die Verwendung das Hinzufügen der Zusammensetzung, die bioverfügbare Kieselsäureverbindungen umfasst, zu dem Wasser, in dem die Wassertiere gehalten werden, umfasst.

13. Verwendung nach einem der voranstehenden Ansprüche, wobei die Verwendung die Schritte des Vermischens der Zusammensetzung, die bioverfügbare Kieselsäureverbindungen umfasst, mit Futter und des Zugebens des Futters zu dem Wasser, in dem die Wassertiere gehalten werden, umfasst.

## Revendications

1. Utilisation d'une composition comprenant un composé d'acide silicique biodisponible dans l'élevage d'animaux aquatiques choisis dans le groupe constitué par les poissons, les crustacés et les mollusques, où l'utilisation a pour objectif et/ou pour résultat un ou plusieurs des effets suivants :
- améliorer la croissance de l'animal aquatique ;
- accélérer la croissance de l'animal aquatique ;
- augmenter le poids de l'animal aquatique ;
- réduire le temps nécessaire à la récolte ;
- améliorer l'utilisation alimentaire ; et
- augmenter le taux de conversion alimentaire.

2. Utilisation selon la revendication 1, où l'espèce aquatique est une espèce de la famille des *salmonidés.*

3. Utilisation selon la revendication 1, où l'espèce aquatique est choisie parmi le groupe constitué des crustacés et des mollusques.

4. Utilisation selon l'une quelconque des revendications précédentes, où au moins 90 % en moles du silicium contenu dans la composition se présente sous la forme d'un composé d'acide silicique biodisponible.

5. Utilisation selon l'une quelconque des revendications précédentes, où le composé d'acide silicique biodisponible est choisi parmi le groupe constitué par l'acide silicique monomère, l'acide silicique dimère, l'acide silicique oligomère et l'acide silicique polymère sous forme subcolloïdale et les combinaisons de ceux-ci.

6. Utilisation selon la revendication 5, où le composé d'acide silicique biodisponible se présente sous la forme de particules subcolloïdales ayant une taille comprise entre 1 et 10 nm, telle que déterminée par spectroscopie RMN ²⁹Si.

7. Utilisation selon la revendication 6, où au moins 50 % des particules contenant de l'acide silicique ont une taille comprise entre 1 et 10 nm.

8. Utilisation selon l'une quelconque des revendications précédentes, où la composition comprend une solution ou une dispersion aqueuse acidifiée de composés d'acide silicique biodisponibles sous forme de particules subcolloïdales, de préférence en combinaison avec de l'acide borique et/ou un additif absorbant l'eau.

9. Utilisation selon l'une quelconque des revendications précédentes, où l'utilisation a pour objectif et/ou pour résultat une augmentation du poids moyen des animaux aquatiques d'au moins 5 %, par rapport au poids moyen des animaux aquatiques obtenu dans les mêmes conditions mais sans le traitement à l'acide silicique.

10. Utilisation selon l'une quelconque des revendications 1 et 3 à 9 dans l'élevage d'une espèce choisie parmi le groupe des crevettes.

11. Utilisation selon l'une quelconque des revendications 1 et 4 à 9 dans l'élevage d'une espèce choisie parmi le groupe des poissons, de préférence choisie parmi le groupe des *cichlidés,* des *cyprinidés* et des *siluridés,* plus préférablement choisie parmi le groupe des tilapias, des rohus, des poissons-chats et des pangas.

12. Utilisation selon l'une quelconque des revendications précédentes, où l'utilisation comprend l'ajout de la composition comprenant des composés d'acide silicique biodisponibles à l'eau dans laquelle les animaux aquatiques sont élevés.

13. Utilisation selon l'une quelconque des revendications précédentes, où l'utilisation comprend les étapes consistant à mélanger la composition comprenant des composés d'acide silicique biodisponibles aux aliments et à ajouter les aliments à l'eau dans laquelle les animaux aquatiques sont élevés.
